# EUROPEAN PATENT SPECIFICATION

(11) **EP 1 335 709 B1**
(45) Date of publication and mention of the grant of the patent: **03.05.2006**
(21) Application number: 01983069.4
(22) Date of filing: 20.11.2001
(51) Int. Cl.: A61K 9/48, A61K 31/4439

(54) **CAPSULE OF CELLULOSE DERIVATIVES SUCH AS HPMC CONTAINING BENZIMIDAZOLE DERIVATIVES SUCH AS OMEPRAZOLE**
ZELLULOSEDERIVATKAPSEL, WIE Z. B. HPMC, DIE BENZIMIDAZOLDERIVAT ENTHAELT, WIE Z.B. OMEPRAZOL
NOUVELLE FORMULATION PHARMACEUTIQUE SOUS FORME DE CAPSULES DE CELLULOSE, ADAPTEE POUR DES DERIVES DE BENZIMIDAZOLE

(30) Priority: 20.11.2000 SI 200000282
(43) Date of publication of application: 20.08.2003
(73) Proprietor: LEK farmacevtska druzba d.d., 1526 Ljubljana (SI)
(72) Inventor: SIRCA, Judita, 1000 Ljubljana (SI)
(74) Representative: Dietz, Jörg-Reimar
(86) International application number: PCT/SI2001/000031
(87) International publication number: WO 2002/039980

(56) References cited:
- EP-A- 0 496 437
- EP-A- 0 960 620
- EP-A- 1 072 258
- WO-A-96/24375
- WO-A-99/03453
- US-A- 5 431 917
- T. OGURA, Y. FURUYA, M. MAATSUURA: "HPMC Capsules-An Alternative to Gelatin" PHARMACEUTICAL TECHNOLOGY EUROPE, vol. 10, no. 11, November 1998 (1998-11), pages 32-42, XP001062392 cited in the application

## Description

### Technical Field of the Invention

The invention belongs to the field of pharmaceutical technology and relates to the use of hard capsules for active substances that have poor water solubility, quickly disintegrate in an acidic medium and are unstable in the presence of moisture, solvents and acidic substances, whereas they have good stability in a neutral or alkaline medium, are quickly absorbed and metabolized and have an extended period of action. More precisely, the present invention relates to the use of hard capsules containing HPMC as the base, for the preparation of a pharmaceutical form with controlled release of active substances which are benzimidazole derivatives in enteric coating pellets filled in said capsule.

### Technical Problem

There exists a constant need for the preparation of pharmaceutical formulations in the form of capsules, by which, in a technologically simple manner, a good stability of active substances that have poor water solubility, quickly disintegrate in an acidic medium and are unstable in the presence of moisture, solvents and acidic substances, whereas they have good stability in a neutral or an alkaline medium, are quickly absorbed and metabolized and have an extended period of action, is achieved. Hitherto known pharmaceutical formulations in the form of capsules containing such active substances use enteric coating pellets filled into hard gelatin capsules for providing a suitable stability of the active substance. These capsules have the disadvantage that they contain 13 to 15 wt. % of water. Therefore an additional drying of the capsules filled with enteric coating pellets is necessary.

### Prior Art

In US 5,264,223 and US 5,431,917 hard capsules containing water-soluble cellulose derivative as the base, a gel-forming agent and a gelling additive are described. An advantage of the described capsules is that they are less brittle in conditions of lower humidity.

In US 5,756,123 hard gelatin capsules containing a water-soluble cellulose derivative such as hydroxypropyhnethyl cellulose (HPMC) as the base are described. The main advantage of the described capsules is the use of the water-soluble cellulose derivative HPMC as the base, which does not disintegrate under special conditions (e.g. after consuming milk products).

In the article of T. Ogura, Y. Furuya, S. Matsuura (Pharmaceutical Technology Europe, November 1998, Volume 10, Number 11, pp. 32-42), a comparison between gelatin and HPMC capsules is described. It is stated that HPMC capsules are, *inter alia,* also suitable for active substances that are unstable in water. For active substances that are extremely sensitive to moisture, however, in order to increase their stability, pharmaceutically acceptable excipients absorbing water have to be added to the pharmaceutical formulation and an additional drying agent to the package nevertheless.

In WO 99/03453 there is described a novel pharmaceutical formulation with controlled release of active substances that are unstable in an acidic medium, unstable at extended storage in the presence of water and at the same time sensitive to heating, which is prepared by anhydrous granulation of active substances and additionally dried pharmaceutically acceptable substances with all pharmaceudcally acceptable excipients dried prior to use in such a manner that their moisture content is lower than 1.0 %, preferably lower than 0.5 %. Enteric coating pellets are then filled into hard gelatin capsules.

EP 960,620-A1, a post-published prior art according to Rule 27(1)(b) EPC, describes an oral pharmaceutical formulation comprising a mixture of a substituted pyridylsulfinyl benzimidazole and a carrier comprising at least one polymer having vinylpyrrolidone monomeric units. The described capsules consist of two essential constituents : a) shell and b) one or more enteric polymers. An enteric polymer is included in the shell or it is in an outer enteric coating which covers the shell (when the shell is of conventional type). Shell can be made of gelatine or HPMC but an enteric coating is essential for the described pharmaceutical composition.

### Description of Solution to Technical Problem with Examples

The invention is based upon the task to prepare a novel pharmaceutical formulation with active substances that have poor water solubility, quickly disintegrate in an acidic medium and are unstable in the presence of moisture, solvents and acidic substances, whereas they have good stability in a neutral or alkaline medium, are quickly absorbed and metabolized and have an extended period of action. In the narrow sense, the invention deals with a pharmaceutical formulation in which enteric coating pellets are filled into hard cellulose capsules consisting of hydroxypropyhmethyl cellulose (HPMC) as the base.

It has surprisingly been found that cellulose capsules are also suitable for active substances that have poor water solubility, quickly disintegrate in an acidic medium and are unstable in the presence of moisture, solvents and acidic substances, whereas they have good stability in a neutral or alkaline medium, are quickly absorbed and metabolized and have an extended period of action. It has to be pointed out that into the inventive pharmaceutical formulation as such, no pharmaceutically acceptable excipient absorbing water has to be added nor does any additional drying agent have to be inserted into the package.

In comparison to hard gelatin capsules, the technological process with cellulose capsules simplifies and shortens the technological process of manufacturing a pharmaceutical formulation according to the invention, since there is no need for any additional drying of already filled capsules to achieve the same stability of the active substances as by using hard gelatin capsules. The process of drying hard gelatin capsules lasts from 15 to 20 hours, which means that during all this time the active substance, which is poorly water-soluble and unstable in the presence of moisture, is exposed to the humid environment of the gelatin capsule.

By the use of cellulose capsules where drying is not necessary, the complete process of manufacturing the pharmaceutical formulation according to the invention is shortened, which is of essential importance with active substances that are poorly water-soluble, quickly disintegrate in an acidic medium and are unstable in the presence of moisture, solvents and acidic substances. Drying is an energy-consuming process, therefore the omission of drying saves large amounts of energy. Additionally, by the use of cellulose capsules instead of hitherto common hard gelatin capsules more production series of the final product can be made within the same period of time.

Pharmaceutical formulation according to the invention, wherein enteric coating pellets are filled into cellulose capsules, is especially suitable for active substances such as benzimidazole derivatives, which are known as substances that have poor water solubility, quickly disintegrate in an acidic medium and are unstable in the presence of moisture, solvents and acidic substances, whereas they have good stability in a neutral or alkaline medium, are quickly absorbed and metabolized and have an extended period of action. As active substances various benzimidazole derivatives can be used such as omeprazole, lansoprazole, timoprazole, rabeprazole, pantoprazole, leminoprazole, pariprazole, esomeprazole and their pharmaceutically acceptable salts such as sodium and magnesium salts. Active substances may also be in the form of their optical isomers and their pharmaceutically acceptable salts such as sodium and magnesium salts. These active substances are known as proton pump inhibitors and are used in the treatment of gastrointestinal diseases.

Enteric coating pellets to be filled in cellulose capsules are prepared according to a process of anhydrous granulation of active substances and of additionally dried pharmaceutically acceptable excipients, which are, prior to use, dried in such a manner that their moisture content is less than 1.0 %, preferably less than 0.5 %. The composition of enteric coating pellets and the manufacture thereof are described in WO 99/03453. Enteric coating pellets may be filled into cellulose capsules on all known capsulating machines that are usually used for filling pellets into hard gelatin capsules.

The invention is illustrated but in no way limited by the following working examples.

### Example 1:

### a) Pellet cores

Composition for 1000 g of pellet cores.

| | |
|---|---|
| Omeprazole | 100 g |
| Low substituted hydroxypropylcellulose (13 to 16 % of hydroxypropoxy groups) | 150 g |
| Microcrystalline cellulose | 150 g |
| Mannitol | 478 g |
| Sodium cross-linked carboxymethylcellulose | 50 g |
| Polyvinylpyrrolidone K 25 | 70 g |
| Polyoxyethylated hydrogenated castor oil | 2 g |

The used pharmaceutically acceptable excipients were, prior to use, dried in such a manner that their moisture content was less than 1.0 %, preferably less than 0.5 %.

A series of 1000 g of pellet cores was prepared according to the following process:

2 g of polyoxyethylated hydrogenated castor oil (Cremophor® RH 40) was dissolved at room temperature in 300 g of absolute ethanol. At room temperature, the formed solution (302 g) was dispersed in a fluidized bed granulator onto previously prepared homogeneous mixture of powder components 100 g of omeprazole, 150 g of dried low-substituted hydroxypropylcellulose (L-HPC LH-20), 150 g of dried microcrystalline cellulose, 478 g of dried mannitol, 50 g of dried sodium cross-linked carboxymethylcellulose and 70 g of dried polyinylpyrrolidone K 25. So prepared plastic mixture was extruded and than spheronized. The formed pellet cores were dried in a fluidized bed or in a chamber dryer at a temperature of inlet air from 35 to 45°C, until the moisture content was less than 0.5 %.

In such a manner 1000 g of pellet cores were obtained.

### b) Enteric coating pellets

| | |
|---|---|
| Pellet cores | 1000 g |
| Hydroxypropylmethyl cellulose phthalate | 150 g |
| Dibutyl sebacate | 15 g |

150 g of hydroxypropylmethyl cellulose phthalate and 15 g of dibutyl sebacate were dissolved at room temperature in a mixture of 1754 g of absolute ethanol and 438 g of acetone. The prepared solution was sprayed onto pellet cores in a fluidized bed apparatus.

### c) Capsulation

On a capsulating machine with gravimetrical filling the manufactured enteric coating pellets were filled into cellulose capsules Qualicaps Shionogi (cellulose capsules containing hydroxypropylmethyl cellulose as the cellulose derivative) with the content of omeprazole amounting to 20 mg/capsule.

### Example 2:

### a) Pellet cores

Composition for 1000 g of pellet cores:

| | |
|---|---|
| Lansoprazole | 100 g |
| Microcrystalline cellulose | 200 g |
| Mannitol | 598 g |
| Sodium starch glycolate | 50 g |
| Polyvinylpyrrolidone K 25 | 50 g |
| Polysorbate 80 | 2 g |

The used pharmaceutically acceptable excipients were, prior to use, dried in such a manner that their moisture content was less than 1.0 %, preferably less than 0.5 %.

Pellet cores were prepared according to the same process as in the Example 1 with exception that the active substance omeprazole was replaced by lansoprazole, dried low-substituted hydroxypropylcellulose (L-HPC LH-20) was replaced by microcrystalline cellulose, sodium cross-linked carboxymethylcellulose was replaced by sodium starch glycolate and the surfactant polyoxyethylated hydrogenated castor oil (Cremophor® RH 40) was replaced by Polysorbate 80.

### b) Enteric coating pellets

| | |
|---|---|
| Pellet cores | 1000 g |
| Eudragit L 100 | 150 g |
| Dibutyl sebacate | 22 g |
| Talc | 15 g |

150 g of Eudragit and 22 g of dibtuyl sebacate were dissolved at room temperature in 1325 g of absolute ethanol and 15 g of talc were dispersed. Under constant stirring the prepared suspension was sprayed onto pellet cores in an apparatus with fluidized air.

### c) Capsulation

On a capsulating machine with gravimetrical filling the manufactured enteric coating pellets were filled into cellulose capsules Qualicaps Shionogi (cellulose capsules containing hydroxypropylmethyl cellulose as a cellulose derivative) with the content of lansoprazole amounting to 20 mg/capsule.

### Example 3

Comparison of pharmaceutical formulations in the form of gelatin capsules and cellulose capsules and advantages of the use of cellulose capsules

### a) Preparation of pharmaceutical formulation

For the comparative test enteric coating pellets were prepared according to the process described in Example 1, points a) and b), whereupon, on a capsulating machine with gravimetrical filling, some were filled into hard gelatin capsules and others into cellulose capsules Qualicaps Shionogi (the cellulose derivative was hydroxypropylmethyl cellulose).

### b) Drying of pharmaceutical formulation

In the technological process of manufacturing the pharmaceutical formulation this phase took place immediately after capsulating.

**Table 1: Comparison of drying time of capsules in the technological process of manufacturing the pharmaceutical formulation**

| Capsules used | Pharmaceutical formulation using hard gelatin capsules | Pharmaceutical formulation using cellulose capsules |
|---|---|---|
| Drying time | 15-20 hours | 0 hours |

### c) Stability of active substance

**Table 2: Comparison of stability of pharmaceutical forms**

| Capsules used | Pharmaceutical formulation using hard gelatin capsules | Pharmaceutical formulation using cellulose capsules |
|---|---|---|
| Related substances and disintegration products after 3 months at conditions of 40 °C and 75% relative humidity | 1.10 % | 1.09 % |

### Determination of related substances and disintegration products

| Principle Chromatography conditions | HPLC determination |
|---|---|
| - stationary phase | Symmetry C8 |
| - dimensions: | 150 x 4.6 mm |
| - temperature: | 30°C or controlled room temperature, |
| Mobile phase: | buffer : acetonitrile |
| | 300 : 100, vol. ratio, |
| Flow: | 0.8 ml/min, |
| Injection volume | 50 µl |
| Wave length | UV, 320 nm. |

From the above tables it is evident that in comparison with the hard gelatin capsules the use of cellulose capsules does not impair the stability of the active substance, yet it brings big savings in the technological process due to the omission of the long drying phase (energy savings, larger production capacity).

A great advantage of the use of cellulose capsules is also the fact that the active substance that is unstable in the presence of moisture is not exposed to the humid environment of the gelatin capsule for a lengthy period of time.

Into the pharmaceutical formulation according to the invention that is manufactured according to the process of anhydrous granulation of the active substance and of dried pharmaceutically acceptable excipients, no pharmaceutically acceptable water-absorbing excipient has to be added. Due to the smaller number of excipients the novel pharmaceutical formulation puts less strain on the patient's organism.

No drying agent has to be added into the final package.

The capsulating of the pharmaceutical formulation with cellulose capsules according to the invention is performed on the same equipment i.e. the same capsulating machines as with hard gelatin capsules.

## Claims

1. A capsule for oral use, **characterized in that** it consists of hydroxypropylmethyl cellulose as the base and that the active substance is a benzimidazole derivative in enteric coating pellets filled in said capsule.

2. A capsule for oral use according to claim 1, **characterized in that** the benzimidazole derivative is selected from a group comprising omeprazole, lansoprazole, timoprazole, rabeprazole, pantoprazole, leminoprazole, pariprazole, esomeprazole, their pharmaceutically acceptable salts, their optically active isomers and pharmaceutically acceptable salts thereof.

3. A capsule for oral use according to claim 2, **characterized in that** the active substance is omeprazole or its pharmaceutically acceptable salt.

4. A capsule for oral use according to claim 2, **characterized in that** the active substance is an optically active isomer of omeprazole or a pharmaceutically acceptable salt thereof.

5. A capsule for oral use according to claim 2, **characterized in that** the active substance is lansoprazole or its pharmaceutically acceptable salt.

6. A capsule for oral use according to claim 2, **characterized in that** the active substance is an optically active isomer of lansoprazole or a pharmaceutically acceptable salt thereof.

7. A pharmaceutical formulation in the form of a capsule for oral use, **characterized in that** it contains:
a) a capsule consisting of hydroxypropylmethyl cellulose as the base and
b) enteric coating pellets filled in said capsule and manufactured by anhydrous granulation of a therapeutically effective amount of an active substance that is a benzimidazole derivative and of dried pharmaceutically acceptable excipients.

8. A pharmaceutical formulation in the form of a capsule for oral use according to claim 7, **characterized in that** the benzimidazole derivative is selected from a group comprising omeprazole, lansoprazole, timoprazole, rabeprazole, pantoprazole, leminoprazole, pariprazole, esomeprazole, their pharmaceutically acceptable salts, their optically active isomers and pharmaceutically acceptable salts thereof.

9. A pharmaceutical formulation in the form of a capsule for oral use according to claim 8, **characterized in that** the active substance is omeprazole or its pharmaceutically acceptable salt.

10. A pharmaceutical formulation in the form of a capsule for oral use according to claim 8, **characterized in that** the active substance is an optically active isomer of omeprazole or a pharmaceutically acceptable salt thereof.

11. A pharmaceutical formulation in the form of a capsule for oral use according to claim 8, **characterized in that** the active substance is lansoprazole or its pharmaceutically acceptable salt.

12. A pharmaceutical formulation in the form of a capsule for oral use according to claim 8, **characterized in that** the active substance is an optically active isomer of lansoprazole or a pharmaceutically acceptable salt thereof.

13. A pharmaceutical formulation in the form of a capsule for oral use according to claim 7, **characterized in that** all used pharmaceutically acceptable excipients are, prior to use, dried in such a manner that their moisture content is less than 1 %.

14. A pharmaceutical formulation in the form of a capsule for oral use according to claim 13, **characterized in that** all used pharmaceutically acceptable excipients are, prior to use, dried in such a manner that their moisture content is less than 0.5 %.

15. A pharmaceutical formulation in the form of a capsule for oral use according to claims 7 to 14, **characterized in that** it is used for the treatment of gastrointestinal diseases.

16. A use of a pharmaceutical formulation in the form of a capsule for oral use for the manufacture of a medicament for the treatment of gastrointestinal diseases, **characterized in that** the pharmaceutical formulation contains:
a) a capsule consisting of hydroxypropylmethyl cellulose as the base and
b) enteric coating pellets filled in said capsule and manufactured by anhydrous granulation of a therapeutically effective amount of an active substance that is a benzimidazole derivative and of dried pharmaceutically acceptable excipients.

17. A use of a pharmaceutical formulation in the form of a capsule for oral use according to claim 16, **characterized in that** the benzimidazole derivative is selected from a group comprising omeprazole, lansoprazole, timoprazole, rabeprazols, pantoprazole, leminoprazole, pariprazole, esomeprazole, their pharmaceutically acceptable salts, their optically active isomers and pharmaceutically acceptable salts thereof.

18. A use of a pharmaceutical formulation in the form of a capsule for oral use according to claim 17, **characterized in that** the active substance is omeprazole or its pharmaceutically acceptable salt.

19. A use of a pharmaceutical formulation in the form of a capsule for oral use according to claim 17, **characterized in that** the active substance is an optically active isomer of omeprazole or a pharmaceutically acceptable salt thereof.

20. A use of a pharmaceutical formulation in the form of a capsule for oral use according to claim 17, **characterized in that** the active substance is lansoprazole or its pharmaceutically acceptable salt.

21. A use of a pharmaceutical formulation in the form of a capsule for oral use according to claim 17, **characterized in that** active substance is an optically active isomer of lansoprazole or a pharmaceutically acceptable salt thereof.

22. A use of a pharmaceutical formulation in the form of a capsule for oral use according to claim 16, **characterized in that** all used pharmaceutically acceptable excipients are, prior to use, dried in such a manner that their moisture content is less than 1 %.

23. A use of a pharmaceutical formulation in the form of a capsule for oral use according to claim 22, **characterized in that** all used pharmaceutically acceptable excipients are, prior to use, dried in such a manner that their moisture content is less than 0.5 %.

## Patentansprüche

1. Kapsel zur oralen Verwendung, **dadurch gekennzeichnet, dass** sie aus Hydroxypropylmethylcellulose als Grundlage besteht und dass der Wirkstoff ein Benzimidazolderivat in enterisch beschichteten Pellets ist, die in diese Kapsel gefüllt sind.

2. Kapsel zur oralen Verwendung nach Anspruch 1, **dadurch gekennzeichnet, dass** das Benzimidazolderivat aus einer Gruppe ausgewählt ist, die besteht aus Omeprazol, Lansoprazol, Timoprazol, Rabeprazol, Pantoprazol, Leminoprazol, Pariprazol, Esomeprazol, ihren pharmazeutisch annehmbaren Salzen, ihren optisch aktiven Isomeren und den pharmazeutisch annehmbaren Salzen hiervon.

3. Kapsel zur oralen Verwendung nach Anspruch 2, **dadurch gekennzeichnet, dass** der Wirkstoff Omeprazol oder dessen pharmazeutisch annehmbares Salz ist.

4. Kapsel zur oralen Verwendung nach Anspruch 2, **dadurch gekennzeichnet, dass** der Wirkstoff ein optisch aktives Isomer von Omeprazol oder ein pharmazeutisch annehmbares Salz hiervon ist.

5. Kapsel zur oralen Verwendung nach Anspruch 2, **dadurch gekennzeichnet, dass** der Wirkstoff Lansoprazol oder dessen pharmazeutisch annehmbares Salz ist.

6. Kapsel zur oralen Verwendung nach Anspruch 2, **dadurch gekennzeichnet, dass** der Wirkstoff ein optisch aktives Isomer von Lansoprazol oder ein pharmazeutisch annehmbares. Salz hiervon ist.

7. Pharmazeutische Formulierung in Form einer Kapsel zur oralen Verwendung, **dadurch gekennzeichnet, dass** sie enthält
a) eine Kapsel, die aus Hydroxypropylmethylcellulose als Grundlage besteht und
b) enterisch beschichtete Pellets, die in diese Kapsel gefüllt sind und mit einer wasserfreien Granulierung einer therapeutisch wirksamen Menge an Wirkstoff, der ein Benzimidazolderivat ist, und getrockneten pharmazeutisch annehmbaren Hilfsstoffen hergestellt sind.

8. Pharmazeutische Formulierung in Form einer Kapsel zur oralen Verwendung nach Anspruch 7, **dadurch gekennzeichnet, dass** das Benzimidazolderivat aus der Gruppe ausgewählt ist, die besteht aus Omeprazol, Lansoprazol, Timoprazol, Rabeprazol, Pantoprazol, Leminoprazol, Pariprazol, Esomeprazol, ihren pharmazeutisch annehmbaren Salzen, ihren optisch aktiven Isomeren und den pharmazeutisch annehmbaren Salzen hiervon.

9. Pharmazeutische Formulierung in Form einer Kapsel zur oralen Verwendung nach Anspruch 8, **dadurch gekennzeichnet, dass** der Wirkstoff Omeprazol oder dessen pharmazeutisch annehmbares Salz ist.

10. Pharmazeutische Formulierung in Form einer Kapsel zur oralen Verwendung nach Anspruch 8, **dadurch gekennzeichnet, dass** der Wirkstoff ein optisch aktives Isomer von Omeprazol oder ein pharmazeutisch annehmbares Salz hiervon ist.

11. Pharmazeutische Formulierung in Form einer Kapsel zur oralen Verwendung nach Anspruch 8, **dadurch gekennzeichnet, dass** der Wirkstoff Lansoprazol oder dessen pharmazeutisch annehmbares Salz ist.

12. Pharmazeutische Formulierung in Form einer Kapsel zur oralen Verwendung nach Anspruch 8, **dadurch gekennzeichnet, dass** der Wirkstoff ein optisch aktives Isomer von Lansoprazol oder ein pharmazeutisch annehmbares Salz hiervon ist.

13. Pharmazeutische Formulierung in Form einer Kapsel zur oralen Verwendung nach Anspruch 7, **dadurch gekennzeichnet, dass** alle verwendeten pharmazeutisch annehmbaren Hilfsstoffe vor der Verwendung auf eine Weise getrocknet werden, dass ihr Feuchtigkeitsgehalt weniger als 1 % beträgt.

14. Pharmazeutische Formulierung in Form einer Kapsel zur oralen Verwendung nach Anspruch 13, **dadurch gekennzeichnet, dass** alle verwendeten pharmazeutisch annehmbaren Hilfsstoffe vor der Verwendung auf eine Weise getrocknet werden, dass ihr Feuchtigkeitsgehalt weniger als 0,5 % beträgt.

15. Pharmazeutische Formulierung in Form einer Kapsel zur oralen Verwendung nach den Ansprüchen 7 bis 14, **dadurch gekennzeichnet, dass** sie zur Behandlung von gastrointestinalen Erkrankungen verwendet wird.

16. Verwendung einer pharmazeutischen Formulierung in Form einer Kapsel zur oralen Verwendung zur Herstellung eines Arzneimittels zur Behandlung von gastrointestinalen Erkrankungen, **dadurch gekennzeichnet, dass** die pharmazeutische Formulierung enthält
a) eine Kapsel, die aus Hydroxypropylmethylcellulose als Grundlage besteht und
b) enterisch beschichtete Pellets, die in diese Kapsel gefüllt sind und mit einer wasserfreien Granulierung einer therapeutisch wirksamen Menge an Wirkstoff, der ein Benzimidazolderivat ist, und getrockneten pharmazeutisch annehmbaren Hilfsstoffen hergestellt sind.

17. Verwendung einer pharmazeutischen Formulierung in Form einer Kapsel zur oralen Verwendung nach Anspruch 16, **dadurch gekennzeichnet dass** das Benzimidazolderivat aus einer Gruppe ausgewählt ist, die besteht aus Omeprazol, Lansoprazol, Timoprazol, Rabeprazol, Pantoprazol, Leminoprazol, Pariprazol, Esomeprazol, ihren pharmazeutisch annehmbaren Salzen, ihren optisch aktiven Isomeren und den pharmazeutisch annehmbaren Salzen hiervon.

18. Verwendung einer pharmazeutischen Formulierung in Form einer Kapsel zur oralen Verwendung nach Anspruch 17, **dadurch gekennzeichnet, dass** der Wirkstoff Omeprazol oder dessen pharmazeutisch annehmbares Salz ist.

19. Verwendung einer pharmazeutischen Formulierung in Form einer Kapsel zur oralen Verwendung nach Anspruch 17, **dadurch gekennzeichnet, dass** der Wirkstoff ein optisch aktives Isomer von Omeprazol oder ein pharmazeutisch annehmbares Salz hiervon ist.

20. Verwendung einer pharmazeutischen Formulierung in Form einer Kapsel zur oralen Verwendung nach Anspruch 17, **dadurch gekennzeichnet, dass** der Wirkstoff Lansoprazol oder dessen pharmazeutisch annehmbares Salz ist.

21. Verwendung einer pharmazeutischen Formulierung in Form einer Kapsel zur oralen Verwendung nach Anspruch 17, **dadurch gekennzeichnet, dass** der Wirkstoff ein optisch aktives Isomer von Lansoprazol oder ein pharmazeutisch annehmbares Salz hiervon ist.

22. Verwendung einer pharmazeutischen Formulierung in Form einer Kapsel zur oralen Verwendung nach Anspruch 16, **dadurch gekennzeichnet, dass** alle verwendeten pharmazeutisch annehmbaren Hilfsstoffe vor der Verwendung auf eine Weise getrocknet werden, dass ihr Feuchtigkeitsgehalt weniger als 1 % beträgt.

23. Verwendung einer pharmazeutischen Formulierung in Form einer Kapsel zur oralen Verwendung nach Anspruch 22, **dadurch gekennzeichnet, dass** alle verwendeten pharmazeutisch annehmbaren Hilfsstoffe vor der Verwendung auf eine Weise getrocknet werden, dass ihr Feuchtigkeitsgehalt weniger als 0,5 % beträgt.

## Revendications

1. Gélule à utilisation orale, **caractérisée en ce qu'**elle consiste en l'hydroxypropylméthylcellulose comme base, et que la substance active est un dérivé de benzimidazole sous forme de pilules à enrobage entérique contenues dans ladite gélule.

2. Gélule à utilisation orale selon la revendication 1, **caractérisée en ce que** le dérivé de benzimidazole est choisi dans un groupe comprenant l'oméprazole, le lansoprazole, le timoprazole, le rabéprazole, le pantoprazole, le léminoprazole, le pariprazole, fésoméprazole, leurs sels pharmaceutiquement acceptables, leurs isomères optiquement actifs et les sels pharmaceutiquement acceptables de ceux-ci.

3. Gélule à utilisation orale selon la revendication 2, **caractérisée en ce que** la substance active est l'oméprazole ou son sel pharmaceutiquement acceptable.

4. Gélule à utilisation orale selon la revendication 2, **caractérisée en ce que** la substance active est un isomère optiquement actif de l'oméprazole ou un sel pharmaceutiquement acceptable de celui-ci.

5. Gélule à utilisation orale selon la revendication 2, **caractérisée en ce que** la substance active est le lansoprazole ou son sel pharmaceutiquement acceptable.

6. Gélule à utilisation orale selon la revendication 2, **caractérisée en ce que** la substance active est un isomère optiquement actif du lansoprazole ou l'un de ses sels pharmaceutiquement acceptables.

7. Formulation pharmaceutique sous forme d'une gélule à utilisation orale, **caractérisée en ce qu'**elle contient :
a) une gélule constituée d'hydroxypropylméthylcellulose comme base, et
b) des pilules à enrobage entérique contenues dans ladite gélule, et préparées par granulation à l'état anhydre d'une quantité thérapeutiquement efficace d'une substance active qui est un dérivé de benzimidazole, et d'excipients anhydres pharmaceutiquement acceptables.

8. Formulation pharmaceutique sous forme d'une gélule à utilisation orale selon la revendication 7, **caractérisée en ce que** le dérivé de benzimidazole est choisi dans un groupe comprenant l'oméprazole, le lansoprazole, le timoprazole, le rabéprazole, le pantoprazole, le léminoprazole, le pariprazole, l'ésoméprazole, leurs sels pharmaceutiquement acceptables, leurs isomères optiquement actifs et les sels pharmaceutiquement acceptables de ceux-ci.

9. Formulation pharmaceutique sous forme d'une gélule à utilisation orale selon la revendication 8, **caractérisée en ce que** la substance active est l'oméprazole ou son sel pharmaceutiquement acceptable.

10. Formulation pharmaceutique sous forme d'une gélule à utilisation orale selon la revendication 8, **caractérisée en ce que** la substance active est un isomère optiquement actif de l'oméprazole ou un sel pharmaceutiquement acceptable de celui-ci.

11. Formulation pharmaceutique sous forme d'une gélule à utilisation orale selon la revendication 8, **caractérisée en ce que** la substance active est le lansoprazole ou son sel pharmaceutiquement acceptable.

12. Formulation pharmaceutique sous forme d'une gélule à utilisation orale selon la revendication 8, **caractérisée en ce que** la substance active est un isomère optiquement actif du lansoprazole ou l'un de ses sels pharmaceutiquement acceptables.

13. Formulation pharmaceutique sous forme d'une gélule à utilisation orale selon la revendication 7, **caractérisée en ce que** tous les excipients pharmaceutiquement acceptables utilisés sont, avant l'utilisation, déshydratés de telle sorte que leur teneur en humidité soit inférieure à 1 %.

14. Formulation pharmaceutique sous forme d'une gélule à utilisation orale selon la revendication 13, **caractérisée en ce que** tous les excipients pharmaceutiquement acceptables utilisés sont, avant l'utilisation, déshydratés de telle sorte que leur teneur en humidité soit inférieure à 0,5 %.

15. Formulation pharmaceutique sous forme d'une gélule à utilisation orale selon les revendications 7 à 14, **caractérisée** de ce qu'on l'utilise pour le traitement de maladies gastrointestinales.

16. Utilisation d'une formulation pharmaceutique sous forme d'une gélule à utilisation orale pour la préparation d'un médicament pour le traitement de maladies gastrointestinales, **caractérisée en ce que** la formulation pharmaceutique contient :
a) une gélule consistant en l'hydroxypropylméthylcellulose comme base, et
b) des pilules à enrobage entérique contenues dans ladite gélule, et préparées par granulation à l'état anhydre d'une quantité thérapeutiquement efficace d'une substance active qui est un dérivé de benzimidazole, et d'excipients anhydres pharmaceutiquement acceptables.

17. Utilisation d'une formulation pharmaceutique sous forme d'une gélule à utilisation orale selon la revendication 16, **caractérisée en ce que** le dérivé de benzimidazole est choisi dans un groupe comprenant l'oméprazole, le lansoprazole, le timoprazole, le rabéprazole, le pantoprazole, le léminoprazole, le pariprazole, l'ésoméprazole, leurs sels pharmaceutiquement acceptables, leurs isomères optiquement actifs et les sels pharmaceutiquement acceptables de ceux-ci.

18. Utilisation d'une formulation pharmaceutique sous forme d'une gélule à utilisation orale selon la revendication 17, **caractérisée en ce que** la substance active est l'oméprazole ou son sel pharmaceutiquement acceptable.

19. Utilisation d'une formulation pharmaceutique sous forme d'une gélule à utilisation orale selon la revendication 17, **caractérisée en ce que** la substance active est un isomère optiquement actif de l'oméprazole ou un sel pharmaceutiquement acceptable de celui-ci.

20. Utilisation d'une formulation pharmaceutique sous forme d'une gélule à utilisation orale selon la revendication 17, **caractérisée en ce que** la substance active est le lansoprazole ou son sel pharmaceutiquement acceptable.

21. Utilisation d'une formulation pharmaceutique sous forme d'une gélule à utilisation orale selon la revendication 17, **caractérisée en ce que** la substance active est un isomère optiquement actif du lansoprazole ou l'un de ses sels pharmaceutiquement acceptables.

22. Utilisation d'une formulation pharmaceutique sous forme d'une gélule à utilisation orale selon la revendication 16, **caractérisée en ce que** tous les excipients pharmaceutiquement acceptables utilisés sont, avant l'utilisation, déshydratés de telle sorte que leur teneur en humidité soit inférieure à 1 %.

23. Utilisation d'une formulation pharmaceutique sous forme d'une gélule à utilisation orale selon la revendication 22, **caractérisée en ce que** tous les excipients pharmaceutiquement acceptables utilisés sont, avant l'utilisation, déshydratés de telle sorte que leur teneur en humidité soit inférieure à 0,5 %.
